# EUROPÄISCHE PATENTSCHRIFT

(11) **EP 2 162 091 B1**
(45) Veröffentlichungstag und Bekanntmachung des Hinweises auf die Patenterteilung: **01.12.2010**
(21) Anmeldenummer: 08784600.2
(22) Anmeldetag: 02.07.2008
(51) Int. Cl.: A61F 2/06, D04B 21/12

(54) **TEXTILE GEFÄSSPROTHESE**
TEXTILE VESSEL PROSTHESIS
PROTHÈSE VASCULAIRE TEXTILE

(30) Priorität: 03.07.2007 DE 102007032156
(43) Veröffentlichungstag der Anmeldung: 17.03.2010
(73) Patentinhaber: Aesculap AG, 78532 Tuttlingen/Donau (DE)
(72) Erfinder: GOLDMANN, Helmut, 78532 Tuttlingen/Donau (DE); MERCKLE, Christof, 68199 Mannheim (DE); PROBST, Dietmar, 37247 Grossalmerode-Trubenhausen (DE); MILWICH, Markus, 73770 Denkendorf (DE); MÜLLER, Erhard, 70565 Stuttgart (DE)
(74) Vertreter: Patentanwälte Ruff, Wilhelm, Beier, Dauster & Partner
(86) Internationale Anmeldenummer: PCT/EP2008/005397
(87) Internationale Veröffentlichungsnummer: WO 2009/003693

(56) Entgegenhaltungen:
- EP-A- 1 498 083
- WO-A-2004/100827
- WO-A-2007/047420
- US-A1- 2006 271 157

## Beschreibung

Die vorliegende Erfindung betrifft eine textile Gefäßprothese auf der Basis von Folienbändchen, welche sich insbesondere für den kleinlumigen Gefäßersatz eignet.

Gefäßprothesen werden in der Regel für den Ersatz von beschädigten oder krankhaft veränderten Arterien eingesetzt. Dazu ist erforderlich, dass das Prothesenmaterial möglichst bioverträgliche und insbesondere biostabile Eigenschaften aufweist. Typischerweise wird Polytetrafluorethylen (PTFE) oder Polyethylenterephthalat (PET) als Prothesenmaterial verwendet.

PTFE verfügt über die erforderliche Bioverträglichkeit und darüber hinaus über hervorragende physikalische Eigenschaften, beispielsweise thermische Stabilität in einem weiten Temperaturbereich. Zudem handelt es sich im Falle von PTFE um ein chemisch inertes Material, welches mühelos zu unterschiedlichen Formerzeugnissen verarbeitet werden kann. Derartige Formerzeugnisse sind beispielsweise aus der EP 0 677 120 B1, EP 0 663 025 B1 und EP 0 391 887 B1 bekannt.

Eine PTFE-Gefäßprothese geht insbesondere aus der US 2006/0271157 A1 hervor. Die dort beschriebene Prothese wird aus kalt verstreckten PTFE-Garnen hergestellt. Kleinlumige und insbesondere dünnwandige Gefäßprothesen werden vor allem für periphere Bypässe sowie für den Gefäßersatz im Fußbereich eingesetzt.

Gefäßprothesen sind auch aus der WO 2004/100827 und der EP 1498083 bekannt.

Aufgabe der vorliegenden Erfindung ist es daher, eine möglichst dünnwandige Gefäßprothese, insbesondere zum Ersatz von kleinlumigen Gefäßen, bereitzustellen, welche über eine gute Offenheitsrate sowie insbesondere flexible Eigenschaften verfügt.

Diese Aufgabe wird gelöst durch eine textile Gefäßprothese auf der Basis von Folienbändchen, wobei die Folienbändchen zumindest teilweise durch längs gerichtete Schlitze eine multifilamentartige Struktur besitzen. Vorzugsweise besitzen alle Folienbändchen der Gefäßprothese durch längs gerichtete Schlitze eine multifilamentartige Struktur.

Die Folienbändchen weisen durch ihre in Längsrichtung erfolgte Schlitzung filamentartige Strukturen auf, wodurch die multifilamentartige Struktur der Folienbändchen erzeugt wird. Die filamentartigen Strukturen können entlang ihrer Längsrichtung an mehreren Stellen miteinander verbunden sein. Insbesondere können die filamentartigen Strukturen ihrerseits in Längsrichtung geschlitzt vorliegen, wodurch feinste Faserstrukturen ausgebildet werden können.

In einer bevorzugten Ausführungsform handelt es sich bei der textilen Gefäßprothese um eine gewebte Prothese. Die in Längsrichtung geschlitzen Folienbändchen lassen sich zum Beispiel mit besonderem Vorteil als Kett- und/oder Schussfäden zu einer erfindungsgemäßen Gefäßprothese weben. Bevorzugt ist die Gefäßprothese durch eine Leinwandbindung hergestellt. In dieser Ausführungsform liegen die Folienbändchen vorzugsweise direkt, d.h. ohne Abstand, aufeinander und insbesondere nebeneinander.

In einer anderen möglichen Ausführungsform handelt es sich bei der textilen Gefäßprothese um eine gewirkte Prothese.

Die Folienbändchen liegen vorzugsweise flach in der textilen Bindung, vorzugsweise der Gewebebindung. Bevorzugt besteht die Gefäßprothese ausschließlich aus den Folienbändchen. Die Folienbändchen können insbesondere in Form von Garnen, beispielsweise texturierten Garnen, vorliegen. Vorzugsweise liegen die Folienbändchen in Form von Glattgarnen vor. Bei den Folienbändchen kann es sich zudem um schrumpffähige Fäden, insbesondere Garne, handeln. Es ist weiterhin bevorzugt, dass die Folienbändchen eine Festigkeit von 80 bis 120 Denier, insbesondere von ca. 100 Denier, aufweisen.

In einer weiteren Ausführungsform verlaufen die längs gerichteten Schlitze parallel zueinander. Die längs gerichteten Schlitze können eine Länge von 1 bis 50 mm besitzen. Vorzugsweise besitzen die Schlitze eine Länge von 2 bis 20 mm, insbesondere 8 bis 14 mm, bevorzugt 10 bis 12 mm. Die längs gerichteten Schlitze sind vorzugsweise mindestens zum Teil gegeneinander längs versetzt angeordnet. Die längs gerichteten Schlitze können grundsätzlich um 0,5 bis 4 cm gegeneinander längs versetzt angeordnet sein. Bevorzugt sind die Schlitze um 1 bis 3 cm, insbesondere um ca. 2 cm, gegeneinander längs versetzt angeordnet. Die längs gerichteten Schlitze enden vorzugsweise innerhalb der Folienbändchen. Die Schlitzlängen können weiterhin innerhalb der Folienbändchen variieren.

In einer weitergehenden Ausführungsform beträgt die Bändchenbreite 100 bis 1.000 µm, insbesondere 400 bis 600 µm, vorzugsweise ca. 500 µm. Weiterhin können die Folienbändchen eine Stärke von 10 bis 200 µm, insbesondere 20 bis 100 µm, vorzugsweise von ca. 50 µm, aufweisen.

In einer bevorzugten Ausführungsform liegt die Zahl der Schlitze bei 4 bis 24, insbesondere 6 bis 20, vorzugsweise 8 bis 12, Schlitzen pro 1 mm Bandbreite.

Die Folienbändchen können bei einer Zugbelastung in ihrer Querrichtung einen Querschnitt aus polygonen, insbesondere sechseckigen, Strukturen besitzen. In einer möglichen Ausführungsform sind die Strukturen rechteckig.

In einer bevorzugten Ausführungsform weist die multifilamentartige Struktur der Folienbändchen jeweils weniger als 10 %, vorzugsweise weniger als 5 %, Filamente mit freien Enden auf. Die Folienbändchen können eine Zugfestigkeit von 5 bis 50 cN/tex, insbesondere 10 bis 20 cN/tex, aufweisen.

Die Gefäßprothese weist in einer weiteren Ausführungsform einen Innendurchmesser zwischen 2 und 38 mm, insbesondere 2 und 10 mm, inbesondere 4 und 6 mm, auf.

Die Gefäßprothese kann insbesondere eine Wandstärke aufweisen, die der doppelten Stärke eines Folienbändchens entspricht. Vorzugsweise weist die Gefäßprothese eine Wandstärke von 0,1 bis 0,25 mm, insbesondere von ca. 0,2 mm, auf. Dadurch ist mit besonderem Vorteil der Ersatz von kleinlumigen Gefäßen, vorzugsweise kleinlumigen Blutgefäßen, insbesondere im Herz- und/oder Fußbereich, möglich. Des Weiteren eignet sich die Gefäßprothese auch zur Verwendung als Hülle für sogenannte Stent-Grafts oder Covered-Stents.

Die Gefäßprothese besteht bevorzugt aus Polytetrafluorethylen (PTFE), insbesondere expandiertem PTFE. Bei dem Polytetrafluorethylen kann es sich insbesondere um nicht verstrecktes Polytetrafluorethylen handeln. Weiterhin kann die Gefäßprothese aus einem Polyester, insbesondere Polyethylenterephthalat (PET) bestehen. Polysulfone als Prothesenmaterial sind ebenso denkbar.

Die erfindungsgemäße Gefäßprothese besitzt in einer weiteren vorteilhaften Ausführungsform knickstabile Eigenschaften. Beispielsweise kann die Gefäßprothese als wendelverstärkte Prothese vorliegen. Die Knickstabilität der Gefäßprothese kann durch Wickeln, insbesondere mit einem Draht oder Faden, oder durch Prägen erreicht werden. Die Gefäßprothese kann insbesondere thermisch fixiert vorliegen. Beispielsweise kann die Gefäßprothese zur thermischen Fixierung bei einer Temperatur zwischen 200 und 280 °C, insbesondere bei ca. 250 °C, behandelt werden. Vorzugsweise weist die Gefäßprothese eine Plissierung auf.

In einer weiteren Ausführungsform liegt die Gefäßprothese imprägniert vor. Typischerweise ist die Gefäßprothese mit einer Imprägnierungsschicht versehen. Als Imprägnierungsmaterialien kommen grundsätzlich synthetische und/oder resorbierbare Materialien in Betracht. Beispielsweise kann es sich bei den lmprägnierungsmaterialien um Materialien handeln, wie sie aus der Herstellung von chirurgischen Nahtmaterialien bekannt sind. Bevorzugt ist die Gefäßprothese mit vernetzter Gelatine imprägniert.

Die vorliegende Erfindung betrifft außerdem die Verwendung von Folienbändchen zur Herstellung einer textilen Gefäßprothese zum Ersatz von kleinlumigen Gefäßen, wobei die Folienbändchen zumindest teilweise durch längsgerichtete Schlitze eine multifilamentartige Struktur besitzen. Bei den zu ersetzenden kleinlumigen Gefäßen handelt es sich in der Regel um Blutgefäße, insbesondere Arterien. Wie bereits erwähnt, eignet sich die Gefäßprothese auch zur Verwendung als Hülle bzw. Ummantelung für sogenannte Stent-Grafts oder Covered-Stents. Bezüglich weiterer Merkmale und Einzelheiten zu der textilen Gefäßprothese und den Folienbändchen wird auf die bisherige Beschreibung Bezug genommen.

Durch die erfindungsgemäße Gefäßprothese wird eine Prothese bereitgestellt, welche sich insbesondere für den Ersatz von kleinlumigen Gefäßen, insbesondere kleinlumigen Blutgefäßen, eignet. Die Gefäßprothese zeichnet sich mit besonderem Vorteil dadurch aus, dass die Gefäßwand sehr dünn gehalten werden kann. Gleichzeitig kann die Gefäßprothese flexible und insbesondere knickstabile Eigenschaften aufweisen. Dadurch ist eine Anpassung der Prothese an unterschiedliche Druckverhältnisse im menschlichen und/oder tierischen Körper möglich, wodurch das Risiko eines Prothesenversagens reduziert werden kann.

Weitere Einzelheiten und Merkmale ergeben sich aus den nachfolgenden Figurenbeschreibungen sowie Beispielen, ohne jedoch die vorliegende Erfindung hierauf zu beschränken.
- Figur 1:: schematische Seitenansicht von Folienbändchen, die mit Hilfe der Leinwandbindung gewebt sind,
- Figur 2:: schematische Draufsicht auf ein längs geschlitztes Folien- bändchen,
- Figur 3:: multifilamentartige Struktur eines Folienbändchens.

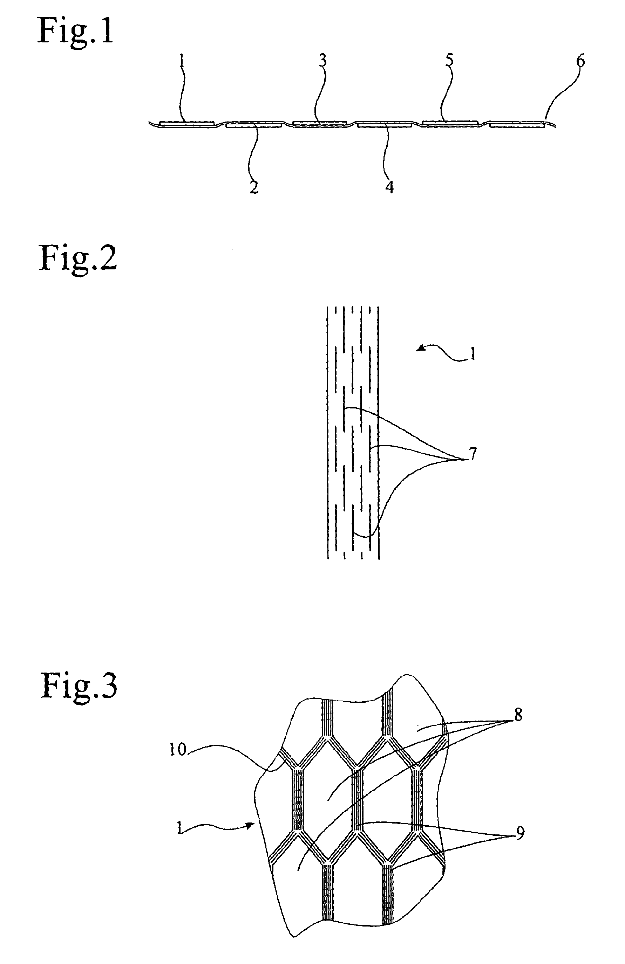

Figur 1 zeigt schematisch eine Leinwandbindung aus Folienbändchen. Dabei kommen die als Kettfäden verwendeten Folienbändchen 1; 2; 3; 4; 5 abwechselnd über und unter dem als Schussfaden verwendeten Folienbändchen 6 zu liegen (1 über 1 unter 1). Die als Schuss- und Kettfäden verwendeten Folienbändchen (1; 2; 3; 4; 5; 6) können bei der Leinwandbindung direkt aufeinander liegen, so dass die Wandstärke einer erfindungsgemäßen Gefäßprothese der zweifachen Stärke eines Folienbändchens entsprechen kann.

Figur 2 zeigt die schematische Draufsicht auf ein zur Herstellung einer erfindungsgemäßen Gefäßprothese verwendbares Folienbändchen 1. Das Bändchen 1 weist in seiner Längsrichtung Schlitze 7 auf. Die Schlitze 7 enden innerhalb des Folienbändchens 1 und sind gegeneinander längs versetzt angeordnet. Dabei sind die Schlitze in Längsrichtung gesehen etwas länger als die zwischen den Schlitzen liegenden ungeschlitzten Stellen.

Figur 3 zeigt schematisch die Struktur eines Folienbändchens 1, wie sie sich bei Zugbelastung des Bändchens 1 in seiner Querrichtung ergeben kann. Das Folienbändchen 1 weist dabei einen Querschnitt aus sechseckigen Lochstrukturen 8 auf, die von den Schlitzen 7 gebildet werden und in Längsrichtung größer sind als in Querrichtung. Die sechseckigen Lochstrukturen 8 werden jeweils durch die in Form von Stegen ausgebildeten filamentartigen Strukturen 9 begrenzt und sind in Längsrichtung gegeneinander versetzt. Die multifilamentartige Struktur des Folienbändchens 1 beruht auf den stegförmig ausgebildeten filamentartigen Strukturen 9. Die Stege 9 können ihrerseits in Längsrichtung Schlitze 10 aufweisen, wobei die längs gerichteten Schlitze 10 vorzugsweise parallel nebeneinander und nicht längs versetzt gegeneinander angeordnet sind.

### Beispiel 1: Herstellung einer gewebten PTFE-Prothese

Zur Herstellung der Prothese werden PTFE-Bändchen, welche in Form von Glattgarnen vorliegen, mit Hilfe von Nadelwalzen in Längsrichtung geschlitzt. Die längs geschlitzten PTFE-Bändchen werden in einen Webstuhl als Kett- und Schussfäden flach eingebracht und mit einer Leinwandbindung zu einer schlauchförmigen Prothese gewebt. Danach wird die Prothese mit einem Draht gewickelt und in einem Ofen bei ca. 220 °C thermisch fixiert. Anschließend wird die Prothese plissiert und mit Gelatine beschichtet, welche in einem nachfolgenden Schritt mit einem Diisocyanat chemisch vernetzt wird. Die hergestellte Gefäßprothese besitzt einen Innendurchmesser von ca. 6 mm und eine Wandstärke von ca. 0,2 mm.

### Beispiel 2: Herstellung einer gewirkten PTFE-Prothese

Zur Herstellung der Prothese werden PTFE-Bändchen, welche in Form von Glattgarnen verwendet werden, durch Nadelwalzen in ihrer Längsrichtung geschlitzt und anschließend zu einer schlauchförmigen Prothese gewirkt. Die gewirkte Prothese wird anschließend geschrumpft, geprägt und in einem Ofen bei ca. 220 °C thermisch fixiert. Danach erfolgt die Plissierung der Gefäßprothese. Anschließend wird die plissierte Gefäßprothese mit Gelatine beschichtet und mit einem Diisocyanat chemisch vernetzt.

## Patentansprüche

1. Textile Gefäßprothese auf der Basis von Folienbändchen, wobei die Folienbändchen zumindest teilweise durch längs gerichtete Schlitze eine multifilamentartige Struktur besitzen.

2. Textile Gefäßprothese nach Anspruch 1, **dadurch gekennzeichnet, dass** die Prothese gewebt ist.

3. Textile Gefäßprothese nach Anspruch 1 oder 2, **dadurch gekennzeichnet, dass** die Folienbändchen in der Gewebebindung flach liegen.

4. Textile Gefäßprothese nach einem der vorhergehenden Ansprüche, **dadurch gekennzeichnet, dass** die Prothese ausschließlich aus den Folienbändchen besteht.

5. Textile Gefäßprothese nach einem der vorhergehenden Ansprüche, **dadurch gekennzeichnet, dass** die Folienbändchen in Form von Glattgarnen vorliegen.

6. Textile Gefäßprothese nach einem der vorhergehenden Ansprüche, **dadurch gekennzeichnet, dass** die längs gerichteten Schlitze parallel verlaufen.

7. Textile Gefäßprothese nach einem der vorhergehenden Ansprüche, **dadurch gekennzeichnet, dass** die längs gerichteten Schlitze mindestens zum Teil gegeneinander längs versetzt angeordnet sind.

8. Textile Gefäßprothese nach einem der vorhergehenden Ansprüche, **dadurch gekennzeichnet, dass** die längs gerichteten Schlitze innerhalb der Folienbändchen enden.

9. Textile Gefäßprothese nach einem der vorhergehenden Ansprüche, **dadurch gekennzeichnet, dass** die Folienbändchen bei Zugbelastung in ihrer Querrichtung einen Querschnitt aus polygonen, insbesondere sechseckigen, Strukturen besitzen.

10. Textile Gefäßprothese nach einem der vorhergehenden Ansprüche, **dadurch gekennzeichnet, dass** die Bändchen eine Zugfestigkeit von 5 bis 50 cN/tex, insbesondere 10 bis 20 cN/tex, aufweisen.

11. Textile Gefäßprothese nach einem der vorhergehenden Ansprüche, **dadurch gekennzeichnet, dass** die Gefäßprothese eine Wandstärke von 0,1 bis 0,25 mm, insbesondere von ca. 0,2 mm, aufweist.

12. Textile Gefäßprothese nach einem der vorhergehenden Ansprüche, **dadurch gekennzeichnet, dass** die Gefäßprothese einen Innendurchmesser zwischen 2 und 38 mm, insbesondere 2 und 10 mm, aufweist.

13. Textile Gefäßprothese nach einem der vorhergehenden Ansprüche, **dadurch gekennzeichnet, dass** die Folienbändchen aus Polytetrafluorethylen bestehen.

14. Textile Gefäßprothese nach einem der vorhergehenden Ansprüche, **dadurch gekennzeichnet, dass** die Gefäßprothese eine Plissierung aufweist.

15. Verwendung von Folienbändchen zur Herstellung einer textilen Gefäßprothese zum Ersatz von kleimlumigen Gefäßen, wobei die Folienbändchen zumindest teilweise durch längsgerichtete Schlitze eine multifilamentartige Struktur besitzen.

## Claims

1. Textile vascular prosthesis based on tape yarns, whereby the tape yarns have, at least in parts, a multifilament-like structure produced by lengthwise-oriented slits.

2. Textile vascular prosthesis according to Claim 1, **characterized in that** the prosthesis is a woven prosthesis.

3. Textile vascular prosthesis according to Claims 1 or 2, **characterized in that** the tape yarns lie flat in the woven construction.

4. Textile vascular prosthesis according to one of the preceding claims, **characterized in that** the prosthesis is made exclusively from the tape yarns.

5. Textile vascular prosthesis according to one of the preceding claims, **characterized in that** the tape yarns are provided in the form of uncrimped yarns.

6. Textile vascular prosthesis according to one of the preceding claims, **characterized in that** the lengthwise-oriented slits run parallel.

7. Textile vascular prosthesis according to one of the preceding claims, **characterized in that** the lengthwise-oriented slits, at least in parts, are arranged in a staggered arrangement in relation to each other in the lengthwise direction.

8. Textile vascular prosthesis according to one of the preceding claims, **characterized in that** the lengthwise-oriented slits end inside the tape yarns.

9. Textile vascular prosthesis according to one of the preceding claims, **characterized in that**, when subjected to a tensile load in their transverse direction, the tape yarns have a cross section in the form of polygonal, especially hexagonal shapes.

10. Textile vascular prosthesis according to one of the preceding claims, **characterized in that** the tape yarns have a tensile strength of 5 to 50 cN/tex, especially 10 to 20 cN/tex.

11. Textile vascular prosthesis according to one of the preceding claims, **characterized in that** the vascular prosthesis has a wall thickness of 0.1 to 0.25 mm, especially of approx. 0.2 mm.

12. Textile vascular prosthesis according to one of the preceding claims, **characterized in that** the vascular prosthesis has an internal diameter of between 2 and 38 mm, especially between 2 and 10 mm.

13. Textile vascular prosthesis according to one of the preceding claims, **characterized in that** the tape yarns are made from polytetrafluoroethylene.

14. Textile vascular prosthesis according to one of the preceding claims, **characterized in that** the vascular prosthesis is pleated.

15. Use of tape yarns to produce a textile vascular prosthesis for replacing small-diameter vessels, whereby the tape yarns, at least in parts, have a multifilament-like structure produced by lengthwise-oriented slits.

## Revendications

1. Prothèse vasculaire textile à base de bandelettes de film, les bandelettes de film ayant du moins partiellement une structure de type multifilament du fait de fentes orientées longitudinalement.

2. Prothèse vasculaire textile selon la revendication 1, **caractérisée en ce que** la prothèse est tissée.

3. Prothèse vasculaire textile selon la revendication 1 ou 2, **caractérisée en ce que** les bandelettes de film sont à plat dans l'assemblage textile.

4. Prothèse vasculaire textile selon une des revendications précédentes, **caractérisée en ce que** la prothèse est composée exclusivement des bandelettes de film.

5. Prothèse vasculaire textile selon une des revendications précédentes, **caractérisée en ce** les bandelettes de film se présentent sous forme de fils lisses.

6. Prothèse vasculaire textile selon une des revendications précédentes, **caractérisée en ce que** les fentes orientées longitudinalement s'étendent parallèlement.

7. Prothèse vasculaire textile selon une des revendications précédentes, **caractérisée en ce que** les fentes orientées longitudinalement sont disposées du moins en partie décalées longitudinalement les unes par rapport aux autres.

8. Prothèse vasculaire textile selon une des revendications précédentes, **caractérisée en ce que** les fentes orientées longitudinalement se terminent dans les bandelettes de film.

9. Prothèse vasculaire textile selon une des revendications précédentes, **caractérisée en ce que** les bandelettes de film, en cas de sollicitation par traction dans leur sens transversal, présentent une section transversale faite de structures polygonales, notamment hexagonales.

10. Prothèse vasculaire textile selon une des revendications précédentes, **caractérisée en ce que** les bandelettes présentent une résistance à la rupture par traction de 5 à 50 cN/tex, notamment 10 à 20 cN/tex.

11. Prothèse vasculaire textile selon une des revendications précédentes, **caractérisée en ce que** la prothèse vasculaire présente une épaisseur de paroi de 0,1 à 0,25 mm, notamment d'environ 0,2 mm.

12. Prothèse vasculaire textile selon une des revendications précédentes, **caractérisée en ce que** la prothèse vasculaire présente un diamètre intérieur compris entre 2 et 38 mm, notamment 2 et 10 mm.

13. Prothèse vasculaire textile selon une des revendications précédentes, **caractérisée en ce que** les bandelettes de film sont composées de polytétrafluoroéthylène.

14. Prothèse vasculaire textile selon une des revendications précédentes, **caractérisée en ce que** la prothèse vasculaire présente un plissage.

15. Utilisation de bandelettes de film pour la fabrication d'une prothèse vasculaire textile en remplacement de vaisseaux à lumen réduit, les bandelettes de film présentant du moins partiellement une structure de type multifilament du fait de fentes orientées longitudinalement.
